# EUROPEAN PATENT APPLICATION

(11) **EP 1 816 195 A1**
(43) Date of publication of application: **08.08.2007**
(21) Application number: 05809735.3
(22) Date of filing: 21.11.2005
(51) Int. Cl.: C12N 15/09, C12Q 1/68, G01N 33/53, C12M 1/00

(54) **NUCLEIC ACID FRAGMENTS FOR DETECTING NUCLEIC ACID AND METHOD OF DETECTING NUCLEIC ACID**

(30) Priority: 19.11.2004 JP 2004335874
(71) Applicant: WAKUNAGA PHARMACEUTICAL CO., LTD., Osaka-shi, Osaka 532-0003 (JP)
(72) Inventor: JIKIHARA, Hiroshi Wakunaga Pharmaceutical Co.,Ltd., Hiroshima 7391105 (JP); KAWAI, Shintaro Wakunaga Pharmaceutical Co., Ltd., Hiroshima 7391105 (JP); OKA, Takanori Wakunaga Pharmaceutical Co., Ltd., Hiroshima 7391105 (JP)
(74) Representative: Ahner, Francis
(86) International application number: PCT/JP2005/021337
(87) International publication number: WO 2006/054740

(57) **Abstract**

A nucleic acid fragment set according to the present invention comprises a plural number of nucleic acid fragments capable of individually hybridizing with a plural number of target sequences, in which each nucleic acid fragment has a region ligatable with each other and the affinity between such ligatable regions is adjusted to a higher level than the affinity between the target sequence and the nucleic acid fragment. The nucleic acid fragment according to the present invention is for use in a nucleic acid fragment kit. The nucleic acid fragment of the present invention can be used as a probe for detecting nucleic acid or a competitor for detecting nucleic acid. According to the present invention, human leukocyte antigen (HLA) genes, T-cell receptor genes, red blood cell group determining genes, and Rh antigen genes, and the like can be detected at a high accuracy level.

## Description

### CROSS-REFERENCE TO RELATED APPLICATIONS

This application is based upon and claims the benefit of priority from the prior Japanese Patent Application No. 2004-335874 (filed on November 19, 2004), the entire contents of which are incorporated herein by reference.

### BACKGROUND OF THE INVENTION

### Field of the Invention

The present invention relates to nucleic acid fragments for detecting mutations or polymorphisms of a nucleic acid of interest in a nucleic acid sample and to a set thereof. Specifically, the present invention relates to a probe for detecting nucleic acid, which is capable of simultaneously recognizing gene information contained in a plural number of discontinued regions present in the same nucleic acid strand, a competitor for detecting nucleic acid, a method for detecting a nucleic acid of interest using them, and a kit for detecting nucleic acid.

### Background Art

Since the entire human genome has been mapped and all human chromosomes have been sequenced, research on information that resides in the individual genes is expected to become more active from now on. Already, research on relationships between a number of various diseases and genetic mutations or polymorphisms has been in progress and relationships between the genetic mutations or polymorphisms and causative genes for single gene diseases or multiple factor diseases have been revealed one after another. Polymorphisms have been reported in many genes and are useful markers for identification of individuals and search for causative genes. Further, it has been reported that compatibility in polymorphisms in ABO red blood cell groups and polymorphisms in HLA genes has an important effect on therapeutic results in blood transfusion or bone marrow transplantation.

Under these circumstances, technology for the simple and accurate detection of genetic mutations and genetic polymorphisms present in a target gene or a region containing the target gene has been desired. Examples of the method for detecting such mutations or polymorphisms are a method for detecting genetic mutations or polymorphisms based on sequence information of the entire fragment of interest, including the PCR-SSCP method (see, for example, Proc. Natl. Acad. Sci. USA. 86, 2766 (1989) (Non-patent Reference No. 1)), the PCR-PHFA method (see, for example, Nucl. Acids Res. 22, 1541- (1994) (Non-patent Reference No. 2)), the PCR-DGGE method (see, for example, Proc. Natl. Acad. Sci. USA. 86, 232 (1989) (Non-patent Reference No. 3)), the PCR-RSCA method, and the sequencing method. In these methods, genetic mutations or polymorphisms present in any region of the fragment can theoretically be found by detecting the difference in the structure of the single strand, the difference in a mode of annealing, the difference in the mode of denaturation, the difference in the structure of heterologous double strand, or the difference generated when a decoded sequence itself is changed.

On the other hand, examples of the method for detecting genetic mutations or polymorphisms in an extremely limited region include the PCR-SSP method, the Invader method, and the PCR-RFLP method. In these methods, genetic mutations or genetic polymorphisms present in an extremely limited region can be found by detecting their effects on the extension reaction from primer ends, the cleavage reaction by a cleavage, and the cleavage reaction by a restriction enzyme.

Further, examples of the in-between method of the above include the PCR-SSO method, the Taqman method, and the LightCycler method. These methods are based on the difference in affinity between a DNA molecule having mutations or polymorphisms in a target region and a synthetic oligonucleotide having a sequence corresponding to the target region. In these methods, genetic mutations or polymorphisms present in the region bound to the synthetic oligonucleotide can be found by detecting the change caused by the change in the affinity with the synthetic oligonucleotide, namely the resultant change in binding of oligonucleotide probes, the cleavage by polymerase, or the presence or absence of fluorescence energy transfer.

The human leukocyte antigen (HLA) have an important role in recognition of self or non-self and a vast number of polymorphisms are present in their genes. The major genes are HLA-A, -B, -C, -DR, -DP, and -DQ genes. By determining the type of these genes of a donor and a recipient prior to transplantation and applying their compatibility, therapeutic effects in bone marrow transplantation and the like can be markedly improved. The number of the kind of polymorphisms for individual genes so far reported reaches from scores to several hundreds. It is not easy to determine alleles of these genes and it is extremely difficult to find a person having a compatible gene type, in particular among unrelated doner, so that bone marrow programs have been publicly organized, accepting a large number of donor registrations to find compatible donors.

Examples of the method for determining the genetic polymorphisms such as for HLA include the PCR-SSP method, the PCR-SSO method, and the direct sequencing method (see, for example, Transplantation Today, vol. 7 Suppl (1994) (Non-patent Reference No. 4)).
Among them, the PCR-SSO method has been most widely used as a suitable method to deal with many samples. In this method, oligonucleotide probes which correspond to a number of domains showing polymorphisms in individual HLA genes are set up and the gene types are determined from their mode of reaction. However, since the human genes consist of two sets of genes, one derived from the mother and the other derived from the father, and moreover the HLA genes are extremely rich in polymorphisms, the gene types occasionally cannot be determined by the PCR-SSO method in which conventional probes are used. For example, in the case where the base sequences at two adjoining positions #1 and #2 of one allele are A and T, respectively, and the base sequences at the corresponding positions of the other allele are T and G, respectively, the bases to be detected by an ordinary method are A and T from position #1 and T and G from position #2. It means that the same bases are ultimately to be detected in the cases where one allele has A and G and the other allele has T and T at the corresponding positions. Namely, the ordinary PCR-SSO method cannot distinguish between the spatially linked and unlinked conditions. Since serial proteins are generally transcribed and translated from serial genes, it is important to know gene sequences in individual allelic units. As mentioned above, polymorphisms cannot be accurately determined if sequences in individual allele units cannot be specified. This also applies to the direct sequencing method.

Saito et al have proposed a probe for detecting nucleic acid which has two specific sequence regions separated each other by nucleotide bases within one probe molecule (see International Patent Publication No. WO 03/027309 (Patent Reference No. 1)). The two specific sequence regions (probe regions to distinguish target sequences) present in this probe are to form a complementary double strand with a target nucleic acid sequence in a sample nucleic acid under hybridization conditions. In the case where the target regions complementary to the two specific regions on the probe are present in the sample nucleic acid on separate strands, the two specific regions are spatially separated from each other on the two strands and the resulting complementary strand becomes a double strand having insufficient strength. However, in the case where the target regions complementary to the two specific regions on the probe are present on the same strand, the resulting complementary strand becomes a double strand having sufficient strength. Therefore, by using the abovementioned probe, the case where the target regions complementary to the two specific regions are spatially separated on two strands and the case where it is spatially adjacent on a single strand can be distinguished based on the strength of the resulting double strand.

However, in order to recognize sequences in two sites by one probe, it is necessary to optimize the sequences at the two sites and to search for the most appropriate combination. For example, in order to simultaneously recognize target sequences present at two sites on the same nucleic acid strand (for example, the number of candidates are m in one site and n in the other site) using a single strand probe, m × n kinds of probes have to be prepared upon optimization of the probe region. If m is 10 and n is 20, 10 × 20=200 kinds of probes have to be prepared and assessed. As a result, the optimization process becomes complicated and the development period has to be extended, which naturally increases the development cost. Under these circumstances, a method in which the probe preparation is easy, the detection accuracy is high, and the operability is excellent has been desired.
* Patent Reference No. 1: International Patent Publication No. WO 03/027309
* Non-patent Reference No. 1: Proc. Natl. Acad. Sci. USA 86, 2766 (1989)
* Non-patent Reference No. 2: Nucl. Acids Res. 22, 1541- (1994)
* Non-patent Reference No. 3: Proc. Natl. Acad. Sci. USA 86, 232 (1989)
* Non-patent Reference No. 4: Implantation Today, vol. 7 SUPPL (1994)

### SUMMARY OF THE INVENTION

The present inventors have recently found that upon preparation of a probe for detecting nucleic acid for the purpose of detecting a nucleic acid of interest having a plural number of target sequences on the same strand, detection sensitivity is equivalent to or better with a probe formed by ligating a plural number of probes with each other via ligatable regions than with the probe having a plural number of serial probe regions on one molecule. The present invention is based on this finding.

Accordingly, an object of the present invention is to provide nucleic acid fragments for use in nucleic acid detection having two or more probe regions with excellent detection accuracy, a set thereof, a probe for nucleic acid detection containing such nucleic acid fragment set, and a method for the detection using such probe.

A nucleic acid fragment set according to the present invention comprises a plural number of nucleic acid fragments capable of individually hybridizing with a plural number of target sequences, in which
each nucleic acid fragment has a region ligatable with each other, and
the affinity between such ligatable regions is adjusted to a higher level than the affinity between the target sequence and the nucleic acid fragment.

A nucleic acid fragment according to the present invention is for use in a nucleic acid fragment set according to the present invention.

A probe for nucleic acid detection according to the present invention comprises a nucleic acid fragment set according to the present invention.
Further, a competitor for nucleic acid detection according to the present invention comprises a nucleic acid fragment set according to the present invention.

According to the present invention, there is provided a method for detecting a nucleic acid of interest in which a plural number of target sequences can be present on the same nucleic acid strand, comprising the steps of
(a) bringing a probe for detecting nucleic acid of claim 7 or 8 into contact with a nucleic acid sample under hybridization conditions, and
(b) assessing the presence of the nucleic acid of interest in the nucleic acid sample by whether or not the probe and the nucleic acid sample are hybridized.

A kit for nucleic acid detection according to the present invention at least comprises a probe for nucleic acid detection according to the present invention and a competitor for nucleic acid detection according to the present invention.
According to the present invention, there is provided use of a nucleic acid fragment set according to the present invention for detecting a nucleic acid of interest in which a plural number of target sequences are present on the same nucleic acid strand.

According another embodiment of the present invention, a nucleic acid fragment according to the present invention can be a nucleic acid fragment in which a plural number of nucleic acid fragments hybridizable with a plural number of target sequences each have a region ligatable with each other and the affinity between such ligatable regions is adjusted to a higher level than the affinity between the target sequence and the nucleic acid fragment.
Further, according to another embodiment of the present invention, there is provided a probe for nucleic acid detection comprising the nucleic acid fragment. According to yet another embodiment of the present invention there is provided a competitor for nucleic acid detection comprising the nucleic acid fragment.

According to another embodiment of the present invention, there is provided a method for detecting a nucleic acid of interest in which a plural number of target sequences are present on the same nucleic acid strand, comprising the steps of (a') preparing a nucleic acid sample, (b') preparing the probe for nucleic acid detection, (c') hybridizing the probe for nucleic acid detection and the nucleic acid sample, and (d') assessing the presence of the nucleic acid of interest in the nucleic acid sample by the absence or presence of a specific hybrid formed between the probe for nucleic acid detection and the nucleic acid sample.

Further, according to another embodiment of the present invention, there is provided a kit for nucleic acid detection, comprising the abovementioned probe for nucleic acid detection and/or a competitor for nucleic acid detection.

Since a probe for nucleic acid detection according to the present invention has a high accuracy in detecting a gene having genetic polymorphisms in a plural number of sites, it can be used for detecting a human leukocyte antigen (HLA) gene, a T-cell receptor gene, a red blood cell group determining gene, an Rh antigen gene, and the like.

### BRIEF DESCRIPTION OF THE DRAWINGS

Fig. 1 is a diagrammatic representation of a probe for detecting nucleic acid according to the present invention (n=2).

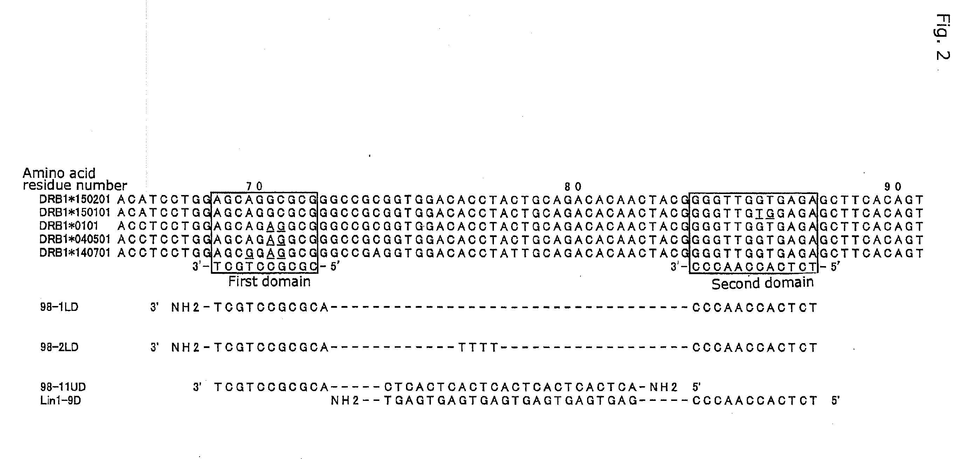

Fig. 2 is a diagram showing the relationship between an HLA-DR gene and a probe for detecting nucleic acid used in Example 1.

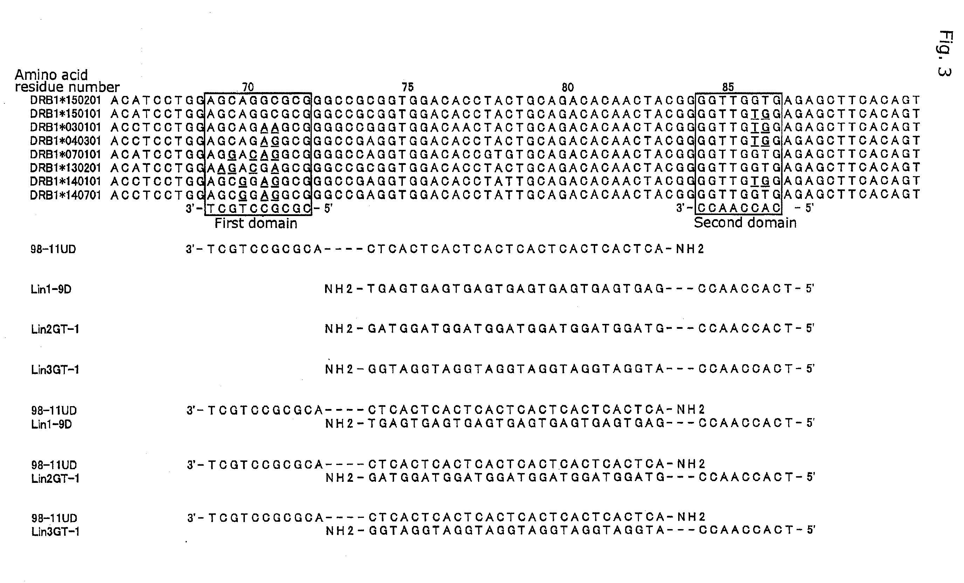

Fig. 3 is a diagram showing the relationship between an HLA-DR gene and a probe for detecting nucleic acid used in Example 2.

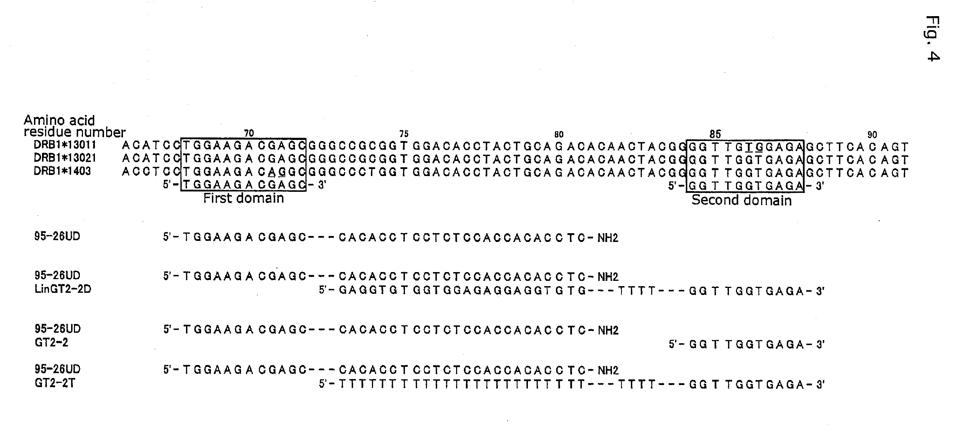

Fig. 4 is a diagram showing the relationship between an HLA-DR gene and a probe for detecting nucleic acid used in Example 3.

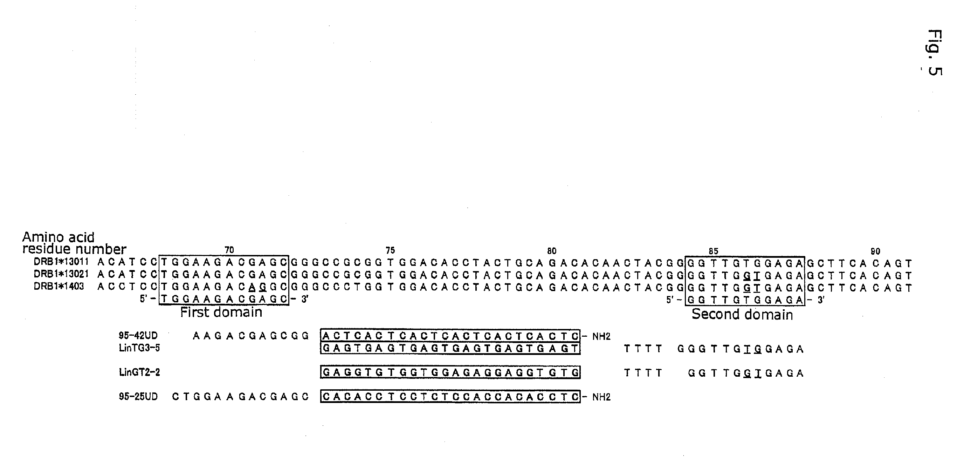

Fig. 5 is a diagram showing the relationship between an HLA-DR gene and a competitor used in Example 4.

### DETAILED DESCRIPTION OF THE INVENTION

### Nucleic acid fragments and sets thereof

A nucleic acid fragment set according to the present invention comprises a plural number of nucleic acid fragments capable of individually hybridizing with a plural number of target sequences, in which each nucleic acid fragment has a region ligatable with each other and the affinity between such ligatable regions is adjusted to a higher level than the affinity between the target sequence and the nucleic acid fragment. And, the nucleic acid fragment according to the present invention is used in the nucleic acid fragment set according to the present invention.

In the present invention, the selection of the target sequence from a nucleic acid of interest can be carried out appropriately based on available genetic information on genetic mutations or polymorphisms.
The term "ligation" herein refers to the formation of bonds between a plural number of molecules in a reaction solution and can be any bonding. The bonds are preferably non-covalent bonds, more preferably hydrogen bonds such as adenine-thymine bonds and guanine-cytosine bonds. The strength of the bonding can be appropriately adjusted according to the length of the sequence and the kind of the nucleic acid in the case of nucleic acids.

In the present invention, the term "affinity" refers to the strength of the bonding between a nucleic acid fragment and a target sequence or between regions ligatable with each other; it can be expressed as a Tm value (melting temperature) in the case between the nucleic acids. The higher the Tm value, the stronger and stabler the bonding is. The Tm value can be determined according to an ordinary method such as those in the following literature.
i) Wallace R.B., Shaffer J., Murphy R.F., Bonner J., Hirose T., and Itakura K. (1979) Hybridization of synthetic oligodeoxyribonucleotides to phi chi 174 DNA: the effect of single base pair mismatch - Nucleic Acids Res. 6(11):3543-3557, or
ii) Breslauer K.J., Frank R., Blocker H., Markey L.A. (1986) Predicting DNA duplex stability from the base sequence - Proc. Natl. Acad. Sci. USA 83, 3746-3750.

In the present invention, the term "hybridization" refers to the formation of a double strand when the complementarity between nucleic acids is high. Generally, nucleic acids are designed to be complementary to each other; however, if necessary, they can contain one or several (for example, 2 to 3) mismatches within the range in which no problem arises upon detection. The number of acceptable mismatches is limited according to the detection accuracy required and the length of the nucleic acid fragment. Upon detection, by appropriately altering the conditions for the hybridization between the nucleic acid fragment and the target nucleic acid, the cases where mismatches are present can be eliminated or the number of acceptable mismatches can be controlled.

### Probes for detecting nucleic acid

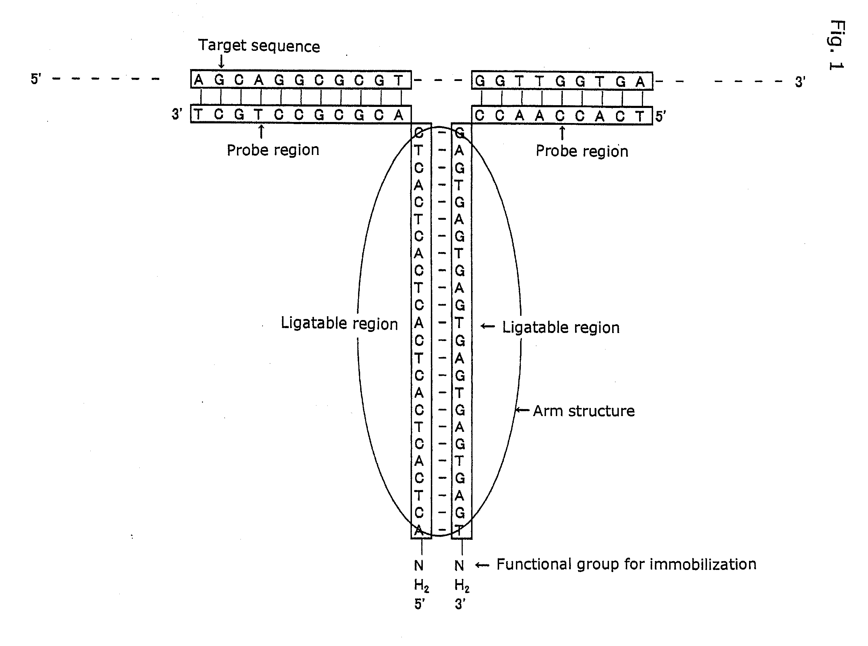

A probe for detecting nucleic acid in the present invention comprises a plural number of nucleic acid fragments capable of individually hybridizing with target sequences in a nucleic acid of interest, in which each nucleic acid fragment has a region ligatable with each other and the affinity between such ligatable regions is adjusted to a higher level than the affinity between the target sequence and the nucleic acid fragment. For example, in the case where the number of the target sequences of the nucleic acid of interest is n and the number of hybridizable nucleic acid fragments used as a probe is n, the regions ligatable with each other exist at least on n-1 sites; and a set of the nucleic acid fragments ligated therein with each other can be used as a probe. Here, n is, for example, 2 to 6, preferably 2 to 4, more preferably 2 to 3, and most preferably 2. An example of the probe can be prepared as shown in Fig. 1.

A nucleic acid fragment in the present invention is not particularly limited as long as it is a nucleic acid specifically bound to a target region of a nucleic acid of interest. Specific examples include DNA (deoxyribonucleic acid), RNA (ribonucleic acid), PNA (peptide nucleic acid), and LNA (locked nucleic acid) and one or more kinds, preferably DNA or PNA, more preferably DNA, can be used. The nucleic acid fragment can be appropriately synthesized by using an ordinary method based on the sequence information for the nucleic acid of interest.

The length of the nucleic acid fragment is not particularly limited and can be, for example, 5 to 100 bases long, preferably 8 to 30 bases long, and more preferably 10 to 28 bases long.

The ligatable regions in the present invention refer to regions which are present on a site different from the site for the nucleic acid fragment capable of hybridizing with a plural number of target sequences and are ligatable with each other by specific affinity. Such regions can be present in the 3' terminus or 5' terminus or both termini of said nucleic acid fragment. Specific examples of the combination include combinations of antigen-antibody, ligand-receptor, and nucleic acid-nucleic acid having a complementary sequence; however, a combination of nucleic acid-nucleic acid having a complementary sequence is preferred.

When the ligatable region is a nucleic acid, its length is typically 4 to 50 bases long, preferably 8 to 40 bases long, and more preferably 10 to 30 bases long. Bases in these combinations can be any sequence, or selected from a group of sequences designed to form base pairs in previously determined combinations. In many cases, it is selected from a series of sequences in which the variation in the sequence is generated by combining sequence units consisting of about 4 bases. The difference in the Tm value (melting temperature) between the ligated probes is higher than that between the target region and the probe region and its temperature difference is more than 2°C, preferably more than 10°C.

A probe according to the present invention can further contain a label, an optional sequence, or a modifying substance which is utilizable by at least one nucleic acid fragment among a plural number of nucleic acid fragments, as long as functions as a probe are not affected.

Further, in a probe for detecting nucleic acid according to the present invention, at least one nucleic acid fragment among a plural number of nucleic acid fragments can be immobilized onto a solid phase. A solid phase carrier used can be made of any material or in any shape as long as the probe for detecting nucleic acid or a sample nucleic acid can specifically be adsorbed onto it or a functional group can be introduced to it to form a covalent bond with the nucleic acid. Specific examples include so-called polymeric microplates, tubes, flat plates, and beads. In the present invention, microplates, flat plates, or beads are preferably used because of their easy mechanization; beads are particularly preferable.

An example of a method for immobilizing a nucleic acid fragment according to the present invention onto a solid phase is the chemical bonding method (Nucleic Acid Res., 15, 5373-5390 (1987)). A specific example is a method in which a carrier having an introduced carboxyl group and a nucleic acid having an introduced aminohexyl group at the 5' terminus are bonded together using a crosslinking agent such as a water-soluble carbodiimide, e.g., EDC.

Another example of the method for immobilization is a method in which a nucleic acid is immobilized directly onto a carrier by non-specific bonding such as adsorption. When the carrier is made of polystyrene, adsorption efficiency can be increased by ultraviolet radiation or the addition of magnesium chloride (Japanese Patent Laid-open Publication No. 1986-219400). Further, another example is a method in which a nucleic acid and a protein are adsorbed to each other via chemical bonding or non-specifically by an appropriate method and the non-specific adsorption of the protein onto a carrier is utilized for immobilization. A subject to be immobilized onto a solid phase can be a sample nucleic acid; a carrier and a method for the immobilization are the same as the case with the probe for detecting nucleic acid according to the present invention.

A probe for detecting nucleic acid according to the present invention is used in detecting a target nucleic acid possibly present in a sample or in identifying a nucleic acid to be tested. The nucleic acid to be detected by a probe according to the present invention can be any nucleic acid as long as it is a nucleic acid of interest having a plural number of target regions on the same strand and preferably a gene derived from a human, more preferably a human leukocyte antigen (HLA) gene, a T-cell receptor gene, a red blood cell group determining gene, or an Rh antigen gene.

Further, such probe is also useful to detect or capture a series of genes having sequences analogous to each other. Occasionally, consecutive common sequences in the base sequences of the series of genes are short so that a stable double strand cannot be formed in such regions by themselves. In such cases, if similar sites are present in adjacent regions, they can be detected or captured by a probe according to the present invention which can simultaneously recognize a plural number of regions.

### Competitors for detecting nucleic acid

Further in the present invention, the abovementioned nucleic acid fragment can be used as a competitor for detecting nucleic acid. When the nucleic acid fragment of the present invention is used as a competitor for detecting nucleic acid, the affinity of a plural number of nucleic acid fragments capable of hybridizing with a plural number of target sequences in a nucleic acid of interest is adjusted to be equivalent to or lower than the affinity when said nucleic acid fragments are used as a probe for detecting nucleic acid. In order to adjust the affinity between the target sequence and the nucleic acid fragment to be equivalent to or lower than the affinity for a probe for detecting nucleic acid, the nucleic acid fragment capable of hybridizing with the target sequence is adjusted to equivalent to the probe or the number of bases capable of hybridizing with the target sequences can be lowered.

In the present invention, a competitor for detecting nucleic acid can be used for the purpose of increasing the accuracy of the hybridization reaction between a nucleic acid of interest and the abovementioned probe for detecting nucleic acid to selectively detect the nucleic acid of interest only. Generally, the amount of the competitor for detecting nucleic acid of the present invention ranges preferably from 1/10 to 200 times, more preferably from 1/2 to 100 times, of the amount of a nucleic acid sample.

Therefore, according to a preferred embodiment of the present invention, in a competitor for detecting nucleic acid, the affinity between a target sequence and a nucleic acid fragment is adjusted to be equivalent to or lower than the affinity between the nucleic acid fragment used in the probe described in claim 7 or 8 and the target sequence.

### Methods for detecting nucleic acid

As mentioned above, according to the present invention, there is provided a method for detecting a nucleic acid of interest in which a plural number of target sequences can be present on the same nucleic acid strand, comprising the steps of
(a) bringing the probe for detecting nucleic acid described in claim 7 or 8 into contact with a nucleic acid sample under hybridization conditions, and
(b) assessing the presence of the nucleic acid of interest in the nucleic acid sample by whether or not the abovementioned probe and the nucleic acid sample are hybridized.

The process for hybridizing a probe for detecting nucleic acid and a nucleic acid sample is not particularly limited, and can be a method in which a nucleic acid sample is hybridized after each probe for detecting nucleic acid is ligated with each other, a method in which a part of probe for detecting nucleic acid is mixed with a nucleic acid sample and then the remaining probe for detecting nucleic acid is added for hybridization, or a method in which a probe for detecting nucleic acid and a nucleic acid sample are each added simultaneously for hybridization.

The conditions under which a probe for detecting nucleic acid and a nucleic acid sample can be hybridized can vary depending on the probe to be used, a target nucleic acid, and the like; however, general conditions can be appropriately established. Specific conditions for hybridization are, for example, in 1 - 5 × SSC (0.75 M NaCl, 75 mM sodium citrate), preferably at a temperature of 25 to 80°C.

As a method for detecting nucleic acid according to the present invention, a commonly used method for general gene detection can be applied. Examples of such method include the following (1) to (5):
(1) a method in which a probe for detecting nucleic acid according to the present invention is previously immobilized onto a solid phase and a nucleic acid of interest having a detectable label or a nucleic acid of interest having a previously introduced label is hybridized with the immobilized probe to detect the label remaining on the solid phase;
(2) a method in which a nucleic acid of interest is previously immobilized onto a solid phase and a probe of the present invention having a previously introduced detectable label is hybridized with the immobilized nucleic acid of interest to detect the label remaining on the solid phase;
(3) a method in which a probe according to the present invention and a nucleic acid of interest are each bonded onto separate suspendable solid phases, an aggregation reaction is induced by hybridizing them, and this aggregation is confirmed.
(4) a method in which a probe according to the present invention and a nucleic acid of interest are hybridized in a liquid phase and fluorescent energy transfer generated by double strand formation is detected; and
(5) a method comprising any combination of the abovementioned (1) to (4).

In the present invention, the method described in (1) above is preferred. In the method described in (1) above, a nucleic acid of interest is easily captured by the immobilized probe of the present invention while no nucleic acid other than the nucleic acid of interest is captured, so that the nucleic acid of interest can be easily detected by measuring the level of the label on the solid phase.

Examples of the method for labelling a probe for detecting nucleic acid according to the present invention or a sample nucleic acid include
(A) a method in which a labelling substance is directly introduced into a nucleic acid to be labelled;
(B) a method in which a nucleic acid to be labelled or a nucleic acid complementary to a nucleic acid to be labelled is synthesized using a labelled oligonucleotide primer; and
(C) a method in which a nucleic acid to be labelled or a nucleic acid complementary to a nucleic acid to be labelled is synthesized using an oligonucleotide primer in the presence of labelled unit nucleic acid.

Examples of the method described in (A) above include a method in which a biotin derivative is introduced by photoreaction into a nucleic acid to be labelled and detection is carried out with an enzyme-binding streptavidin (Nucleic Acids Res., 13, 745 (1985)) and a method in which a nucleic acid to be labelled is sulfonated and detected using an enzyme-labelled anti-sulfonated antibody (Proc. Natl. Acad. Sci. USA, 81, 3466-3470 (1984)).

As the methods described in (B) and (C) above, a method for amplifying a specific nucleic acid sequence (BIO/TECHNOLOGY, 8, 291 (1990)) can be used. For example, in a PCR method (Science, 230, 1350-1354 (1985)), a labelled elongation product or amplification product can be obtained using a labelled primer or a labelled mononucleotide triphosphate. Further, in an amplification method using Qβ replicase (BIO/TECHNOLOGY, 6, 1197 (1988)), a labelled elongation product or amplification product can similarly be obtained using a labelled mononucleotide triphosphate. Further, in nucleic acid amplification methods other than the abovementioned methods, an elongation product or amplification product can be labelled by previously labelling a mononucleotide triphosphate or an oligonucleotide to be incorporated by elongation reaction or amplification reaction.

The labelling substance to be used here can be either radioactive or nonradioactive as long as it can be detected after the hybridization process. A nonradioactive label is preferred from the viewpoint of easy handling, storage, disposal, and the like.

Examples of the nonradioactive label include haptens such as biotin, a 2,4-dinitrophenyl group, and digoxigenin; fluorescein and its derivatives (e.g., fluorescein isothiocyanate (FITC)), rhodamine and its derivatives (e.g., tetramethyl rhodamine isothiocyanate (TRITC) and Texas red), fluorescent substances such as 4-fluoro-7-nitrobenzofurazan (NBDF) and dansyl; and chemiluminescent substances such as acridine. When a target nucleic acid or a probe according to the present invention is labelled by these substances, labelling can be carried out by a known means (see Japanese Patent Laid-open Publication No. 1984-93098 and Japanese Patent Laid-open Publication No. 1984-93099).

In the present invention, the method for detecting the presence or absence of a specific hybrid formed between a probe according to the present invention and a target nucleic acid is appropriately selected and determined depending on the kind of label used.

When the label is directly detectable, namely when the label is, for example, a radioisotope, fluorescent substance, or pigment, the detection can be performed on a labelled nucleic acid bonded onto a solid phase or, alternatively, the label bonded to the nucleic acid or the label detached from the nucleic acid is released in a solution and then the detection can be performed by a method suitable for the label. Further, when the label is indirectly detectable, namely when the label is, for example, a ligand for a specific binding reaction, such as biotin or hapten, the detection can be performed using a receptor (for example, avidin or antibody) to which a label directly generating a signal or an enzyme catalyzing a signal generating reaction is bonded, as generally used for their detection.

According to a preferred embodiment of the present invention, the detection method according to the present invention further comprises the step of amplifying a sample nucleic acid in the sample prior to step (a). Namely, in the detection method according to the present invention, it is preferred to amplify the sample nucleic acid by a common gene amplification method using the sample nucleic acid as a template, prior to the detection. Examples of such amplification method include a method in which a change in temperature is necessary, such as the PCR method (polymerase chain reaction), and a method which is carried out at a constant temperature, such as the LAMP method (loop mediated amplification).

According to another preferred embodiment of the present invention, a competitor for detecting nucleic acid according to the present invention is further used in step (a). The detection accuracy can be improved by using the competitor.

A detecting method of the present invention is used in detecting a target nucleic acid possibly present in a sample or in identifying a nucleic acid to be detected. The nucleic acid to be detected according to the present invention can be any nucleic acid as long as it is a target nucleic acid having a plural number of target regions on the same strand and preferably a gene derived from a human, more preferably a human leukocyte antigen (HLA) gene, a T-cell receptor gene, a blood cell group determining gene, or an Rh antigen gene.

### Kits for detecting nucleic acid

A nucleic acid for detecting nucleic acid according to the present invention is for detecting the presence of a target sequence of a nucleic acid of interest in a sample nucleic acid, at least comprising a probe for detecting nucleic acid according to the present invention and a competitor for detecting nucleic acid according to the present invention.

The kit according to the present invention preferably further comprises a primer to amplify the nucleic acid of interest. Further, the kit according to the present invention can contain a reagent necessary to perform gene amplification reaction, a reagent to carry out hybridization reaction, further a reagent necessary to assess whether or not a gene amplification product and a probe according to the present invention are specifically hybridized, and the like. These reagents are for common use and can be appropriately used. For example, intercalators that specifically react with double strands can be used as reagents to assess whether or not hybridization has occurred.

A kit according to the present invention is used in detecting a target nucleic acid possibly present in a sample or in identifying a nucleic acid to be detected. The nucleic acid to be detected according to the present invention can be any nucleic acid as long as it is a target nucleic acid having a plural number of target regions on the same strand and preferably a gene derived from a human, more preferably a human leukocyte antigen (HLA) gene, a T-cell receptor gene, a red blood cell group determining gene, or an Rh antigen gene.

### EXAMPLES

The present invention will be explained by the following examples that are not intended as a limitation of the invention.

### Example 1: Distinguishment of HLA-DPB1*150201 from HLA-DRB1*150101, *0101, *040501, and *140701

A probe for detecting nucleic acid of the present invention was used for the distinguishment of HLA-DPBL genes (HLA-DRB1*150201 (SEQ ID NO: 1) from HLA-DRB1*150101 (SEQ ID NO: 2), *0101 (SEQ ID NO: 3), *040501 (SEQ ID NO: 4), and *140701 (SEQ ID NO: 5)).

As probes for detecting nucleic acid, probes 98-1LD (SEQ ID NO: 6), 98-2LD (SEQ ID NO: 7), and 98-11UD (SEQ ID NO: 8)/Linl-9D (SEQ ID NO: 9) were prepared. They have probe sequences which recognize a domain corresponding to amino acid residues 69 to 72 (first probe domain) and a domain corresponding to amino acid residues 84 to 88 (second probe domain) on HLA-DRB1*150201 gene.
In probe 98-1LD, the two probe regions have no interruption between them and are continued. In 98-2LD, four thymidylic acid residues (TTTT) are linked between the two domains. In 98-11UD/Lin1-9D, the probe regions corresponding to the two domains are respectively contained in 98-11UD and Linl-9D. Further, 98-11UD has 6 ACTC units at the 5' side of the probe region and Lin1-9D has 6 GAGT units at the 3' side of the probe sequence. These two probes can create an arm structure by forming a complementary double strand at these sites and thus the two probe regions become adjoined through the arm structure. These probes were immobilized onto carboxylated polystyrene beads through amino acid residues present at the end of oligonucleotides by coupling using a water-soluble carbodiimide such as EDC.

On the other hand, as for HLA-DRB1*150201 and *150101 genes, a PCR reaction (30 cycles consisting of 94°C for 30 seconds, 55°C for 30 seconds, and 72°C for 30 seconds) was performed with biotin-labelled primers using plasmids, in which each gene was incorporated, as templates, to obtain amplicons. Further, as for samples #1 to #5, which were previously assessed to have HLA-DRB1 genes as shown in Table 2, amplicons were obtained using chromosomal DNAs. Biotin-labelled amplicons obtained for individual genes were each hybridized with immobilized probes under hybridization conditions. Streptavidin-phycoerythrin (a fluorescent substance) was allowed to react herein and the fluorescence intensity remaining on the beads after washing was measured. Results for the relative values are shown in Table 2.

**Table 1**

| Number of mismatches with DRB1 genes in individual probes prepared in Example 1 | | |
|---|---|---|
| | Number of mismatches | |
| | In the first domain | In the second domain |
| *150201 | 0 | 0 |
| *150101 | 0 | 2 |
| *0101 | 2 | 0 |
| *040501 | 2 | 0 |
| *140701 | 3 | 0 |

**Table 2**

| Fluorescence intensity remaining on the beads | | | | | |
|---|---|---|---|---|---|
| | | | 98-11UD/ Linl-9D | 98-1LD | 98-2LD |
| Sample #1 | *150201 | *0101 | 6140 | 8380 | 2152 |
| Sample #2 | *150101 | *040501 | 136 | 1480 | 189 |
| Sample #3 | *0101 | *040501 | 170 | 858 | 162 |
| Sample #4 | *150101 | *140701 | 43 | 205 | 98 |
| Sample #5 | *150201 | *140701 | 3877 | 6255 | 1130 |
| Plasmid | *150101 | | 95 | 160 | 103 |
| Plasmid | *150201 | | 8230 | 8430 | 4380 |

Table 1 shows the number of mismatches in the first domain and second domain sequences between the probes used and individual DRB1 genes. The probe sequences used in this example are completely matched with DRB1*150201 gene in both two domains but mismatches in either one of the domains are present with other genes.

Table 2 shows the fluorescence intensity remaining on the beads after hybridization using the biotin-labelled DNAs prepared from individual template DNAs. It shows that the probe of the present invention recognizes both the first domain and the second domain as evident from the experimental example using plasmids as template DNAs. Namely, while the signal was strong with DRB1*150201 in which no mismatch is present in both the first and second domain, the signal was weak with DRB1*150101 in which mismatches are present in the second domain. As for the degree of distinguishability, the probe of the present invention was equivalent to or better than the two kinds of probes used for comparison.

Further, it was also revealed that when chromosomal DNAs were used as samples, both the probe of the present invention and the probes for comparison showed a strong signal with the samples containing DRB1*150201 while the signal was weak with the samples without this gene.

In the probe of the present invention, the first domain and the second domain are designed to locate on different molecules and to be ligated by ligatable regions. As shown in this example, it is understood that when a probe having such structure recognizes base sequences at two sites and both sequences are matched similarly to the probes having other shapes in the example, such base pairs become stabler so as to be specifically detectable.

### Example 2: Distinguishment of HLA-DRB1*150201 from DRB1*150101, *030101, *040301, *070101, *130201, *140101, and *140701

Probes for detecting nucleic acid of the present invention were used for distinguishing HLA-DRB1 genes (HLA-DRB1*150201, *150101, *030101 (SEQ ID NO: 10), *040301 (SEQ ID NO: 11), *070101 (SEQ ID NO: 12), *130201 (SEQ ID NO: 13), *140101 (SEQ ID NO: 14), and *140701).

Probes for detecting nucleic acid used were four kinds of probes, i.e., 98-11UD, Lin1-9D, Lin2GT-1 (SEQ ID NO: 15), and Lin3GT-1 (SEQ ID NO: 16), which were individually immobilized; a mixture of 98-11UD and Lin1-9D; a mixture of 98-11UD and Lin2GT-1; and a mixture of 98-11UD and Lin3GT-1.
Here, 98-11UD has a probe region corresponding to amino acid residues 69 to 72 (first domain) and has 6 units of ACTC on its 5' side. Lin1-9D has a probe region corresponding to amino acid residues 84 to 88 (second domain) and has 6 units of GAGT on its 5' side. Lin2GT-1 has a probe region for the second domain and has 6 units of GTAG on its 5' side. Lin3GT-1 has a probe region for the second domain and has 6 units of ATGG on its 5' side. Of these probes, in the combination of 98-11UD and Lin1-9D, their unit sequences are complementary to each other so that they can form an arm structure by ligation.

In the same manner as in Example 1, the probes were immobilized on carboxy beads individually or as a mixture of two kinds. Further, the PCR amplification was carried out with biotin-labelled primers using plasmids or chromosomal DNAs as templates to obtain biotin-labelled PCR products.

The biotin-labelled amplicons obtained for individual genes were hybridized with the beads, onto which individual probes were immobilized, under hybridization conditions. Streptavidin-phycoerythrin (a fluorescent substance) was allowed to react herein and the fluorescence intensity remaining on the beads after washing was measured and its relative values are shown in Table 4.

**Table 3**

| Number of mismatches with DRB1 genes in individual probes prepared in Example 2 | | |
|---|---|---|
| | Number of mismatches | |
| | In the first domain | In the second domain |
| *150201 | 0 | 0 |
| *150101 | 0 | 2 |
| *030101 | 2 | 2 |
| *040301 | 2 | 2 |
| *070101 | 4 | 0 |
| *130201 | 4 | 0 |
| *140101 | 3 | 2 |
| *140701 | 3 | 0 |

**Table 4**

| Fluorescence intensity remaining on the beads | | | | | | | |
|---|---|---|---|---|---|---|---|
| | 98-11UD | 98-11UD/ Lin1-9D | 98-11UD/ Lin2GT-1 | 98-11UD/ Lin3GT-1 | Linl-9D | Lin2GT-1 | Lin3GT-1 |
| Plasmid DRB1*030101 | 58 | 27 | 96 | 90 | 68 | 73 | 85 |
| Plasmid DRB1*040301 | 838 | 25 | 904 | 822 | 70 | 74 | 85 |
| Plasmid DRB1*070101 | 378 | 66 | 468 | 390 | 99 | 90 | 81 |
| Plasmid DRB1*130201 | 104 | 33 | 120 | 100 | 105 | 120 | 122 |
| Plasmid DRB1*140101 | 235 | 22 | 221 | 236 | 74 | 70 | 87 |
| Plasmid DRB1*140701 | 91 | 36 | 79 | 78 | 95 | 104 | 159 |
| Plasmid DRB1*150101 | 262 | 29 | 317 | 291 | 103 | 82 | 109 |
| Plasmid DRB1*150201 | 155 | 1572 | 455 | 364 | 109 | 115 | 126 |
| Sample #1 150201 101 | 814 | 6307 | 3086 | 2399 | 175 | 184 | 216 |
| Sample #2 150201 410 | 514 | 4686 | 2609 | 1830 | 110 | 107 | 123 |
| Sample #3 150101 70101 | 88 | 37 | 124 | 107 | 80 | 81 | 110 |
| Sample #4 1405 130201 | 126 | 44 | 144 | 127 | 96 | 94 | 98 |

Table 3 shows the number of mismatches in the sequences of the first domain and the second domain between the probes used and individual DRB1 genes. The sequences of the probe region used in this example are completely matched with DRB1*150201 gene in both two domains but mismatches are present with other genes in at least one of the domains.

98-11UD having a probe region corresponding to the first domain has a sequence completely matched with DRB1*150101 and *150201 and mismatches with other genes. When this probe was used for chromosomal DNAs as samples, relatively high signals were obtained with samples #1 and #2 which contain a sequence completely matched with *150201 gene. However, as for samples which were amplified using plasmids as templates, signals were higher with DRB1*040301 and *070101, which contain mismatches, than with DRB1*150201, which has a completely matched sequence; thus, the primary functions as a probe cannot be recognized.

Lin1-9D, Lin2GT-1, and Lin3GT-1 each having a probe sequence corresponding to the second domain, have a sequence completely matched with DRB1*070101, *130201, and *150201 and 2-base mismatches with *030101, *040301, *140701, and *150101. These 3 kinds of probes are identical in the sequence of the probe region and different only in the sequences of their units. These probes all showed no difference in signals between genes having complete matches and genes having mismatches.

From these results, it seems to be impossible to simultaneously recognize the first domain and the second domain with a probe molecule having only one of these two domains.

On the other hand, when 98-11UD/Linl-9D, 98-11UD/Lin2GT-1, and 98-11UD/Lin3GT-1, in which probe regions corresponding to the two domains are simultaneously immobilized, were used as probes and chromosomal DNAs were used as samples, high signals were obtained with chromosomal DNA samples #1 and #2 which contain *150201 having complete matches in both two domains and low signals were obtained with samples #3 and #4 which do not contain *150201. Here, distinguishment was more obvious with the combination 98-11UD/Lin1-9D, in which the sequences of the unit parts contained in the two probes are complementary, than with the other two without such complementary sequences.

When plasmid DNAs were used as templates, probe 98-11UD/Linl-9D gave specific signals only with DRB1*150201 having complete matches in the two domains. However, as seen with probe 98-11UD, the other two probes gave strong signals with DRB1*040301 and *070101, which have mismatches; thus, the expected functions as a probe cannot be recognized.

From the results above, a probe in which the two domains are connected with complementary units probably forms a structure having the two domains adjoined via an arm structure, which might result in high distinguishability of the probe to simultaneously recognize the two domains.

### Example 3: Distinguishment of HLA-DRB1*130201 from HLA-DRB1*130101 and HLA-DRB1*1403

Probes for detecting nucleic acid of the present invention were used for distinguishing HLA-DRB1 *130201, HLA-DRB1*130101 (SEQ ID NO: 17), and HLA-DRB1*1403 (SEQ ID NO: 18).

As probes for detecting nucleic acid, 95-26UD (SEQ ID NO: 19) which recognizes only one of the domains was immobilized, and LinGT2-2D (SEQ ID NO: 20), GT2-2 (SEQ ID NO: 21), or GT2-2T (SEQ ID NO: 22), which recognizes the other domain, was added herein upon hybridization. Here, 95-26UD has a probe region corresponding to amino acid residues 69-72 (first domain) and has a Tag sequence 5'-CACACCTCCTCTCCACCACACCTC-3' (SEQ ID NO: 23) on its 3' side. LinGT2-2D has a probe region corresponding to amino acid residues 84-88 (second domain) and has a Tag sequence 5'-GAGGTGTGGTGGAGAGGAGGTGTG-3' (SEQ ID NO: 24) and four thymidylic acid residues on its 5' side. GT2-2 has a probe region of the second domain but has no sequence on its 5' side. GT2-2T has a probe region of the second domain and has a 28-base long sequence consisting of thymidylic acid residues on its 5' side. Among these probes, in the combination of 95-26UD and LinGT2-2D, their Tag sequences are complementary to each other so that an arm structure can be formed by complementary double strand formation by adding LinGT2-2D to the carrier on which 95-26UD is immobilized, which results in a structure in which the first domain recognizing part and the second domain recognizing part are adjoined via the arm part. On the other hand, in the combination of 95-26UD and GT2-2 or 95-26UD and GT2-2T, an arm structure formation by complementary double strand formation cannot occur, which does not result in a structure in which the first domain recognition part and the second domain recognition part are adjoined.

In the same manner as in Example 1, 95-26UD alone was immobilized onto carboxy beads. Further, biotin-labelled amplicons were prepared by PCR amplification with biotin-labelled primers using plasmid DNAs into which individual genes were incorporated, as a template.

The biotin-labelled amplicons obtained for individual genes were hybridized with the beads, onto which 95-26UD was immobilized, under hybridization conditions. LinGT2-2D, GT2-2, or GT2-2T was allowed to coexist herein in the liquid phase. Streptavidin-phycoerythrin (a fluorescent substance) was allowed to react herein and the intensity of fluorescence remaining on the beads after washing was measured. Its relative values are shown in Table 6.

**Table 5**

| Number of mismatches with DRB1 genes in individual probes prepared in Example 3 | | |
|---|---|---|
| | Number of mismatches | |
| | In the first domain | In the second domain |
| *130201 | 0 | 0 |
| *130101 | 0 | 2 |
| *1403 | 2 | 0 |

**Table 6**

| Fluorescence intensity remaining on the beads | | | | |
|---|---|---|---|---|
| Oligonucleotide immobilized | 95-26UD | | | |
| Oligonucleotide added | - | LinGT2-2D | GT2-2 | GT2-2T |
| Plasmid DRB1*130201 | 142 | 3039 | 141 | 141 |
| Plasmid DRB1*130101 | 117 | 46 | 138 | 135 |
| Plasmid DRB1*1403 | 9 | 11 | 35 | 29 |

Table 5 shows the number of mismatches in the sequences of the first domain and the second domain between the probes used and the individual DRB1 genes. The sequences of the probe region used in this example are completely matched with DRB1*130201 gene in both two domains but mismatches are present in the second domain with DRB1*130101 and in the first domain with DRB1*1403.

Table 6 shows the fluorescence intensity remaining on the beads after hybridization using the biotin-labelled amplicons prepared from individual template DNAs. It shows that both the first domain and the second domain were recognized only when a probe, which is capable of forming a structure having the first domain and the second domain being adjoined by complementary double strand formation with the immobilized probe, was added during the hybridization. Namely, when LinGT2-2D, which has a unit complementary to the immobilized probe 95-26UD and can form a structure having the two domains adjoined by complementary double strand formation, was mixed during hybridization, a strong signal was obtained with DRB1*130201 having mismatches neither in the first domain nor in the second domain and weak signals were obtained with DRB1*130101 and DRB1*1403 which have mismatches in one of the domains. On the other hand, when GT2-2 and GT2-2T, in which the sequence of the second domain is the same but an arm structure cannot be formed with the immobilized probe to form a structure with the two domains adjoined, were each added during hybridization, the fluorescence intensity was as low as that without the addition.

In the probe of the present invention, the first domain and the second domain are designed to locate on different molecules and to be adjoined with each other by ligatable regions. As shown in this example, it is understood that also in the case where only one of the domains is immobilized and the other domain is added to a liquid phase, when the base sequences of the two domains are individually matched and the probe can form a structure to have the two domains adjoined, the formed base pairs become stabler so as to be specifically detectable.

### Example 4: Improvement of ability of distinguishment of HLA-DRB1*130101 from DRB1*130201 and DRB1*1403 by the addition of nucleic acid fragments

Using a nucleic acid fragment of the present invention as a competitor, HLA-DRB1 genes (HLA-DRB1*130101, DRB1*130201, and DRB1*1403) were distinguished.

As a method for specifically detecting a nucleic acid sequence, a method in which a nucleic acid having a sequence homologous to a probe or sample sequence is allowed to coexist for the purpose of improving its specificity is well known. A nucleic acid fragment of the present invention is also expected to have the similar function.

As a probe for detecting nucleic acid, a nucleic acid fragment designated according to the present invention was immobilized on a solid phase. Herein, another nucleic acid fragment according to the present invention was added in a liquid phase to assess whether the hybridization specificity can be improved by allowing it to compete with a sample for the binding to the probe.

In this example, a probe recognizing one of the domains was previously immobilized onto a solid phase and another probe recognizing the other domain was added upon hybridization; however, it is also possible to simultaneously immobilize both probes. Further, at the same time, a probe recognizing one domain and a probe recognizing the other domain, which have regions ligatable with each other, were simultaneously added for ligation upon hybridization; however, it is also possible to add them in a previously ligated form. 95-42UD (SEQ ID NO: 25) recognizing the first domain only was immobilized and LinTG3-5 (SEQ ID NO: 26) recognizing the second domain only was added upon hybridization to prepare a probe recognizing the two different domains. Further, as a competitor, 95-25UD (SEQ ID NO: 27) recognizing the first domain, LinGT2-2D recognizing the second domain, or both together was added upon hybridization. Here, 95-42UD has a probe region corresponding to amino acid residues 69 to 72 (first domain) and has a Tag sequence consisting of 6 units of ACTC on its 3' side. LinTG3-5 has a probe region corresponding to amino acid residues 84 to 88 (second domain) and has a Tag sequence consisting of 6 units of GACT and 4 units of thymidylic acid on its 5' side. Their Tag sequences are complementary to each other and can be easily ligated when mixed. 95-25UD used as a competitor has a probe region corresponding to amino acid residues 69 to 72 (first domain) and has a Tag sequence 5'-CACACCTCCTCTCCACCACACCTC-3' (SEQ ID NO: 23) on its 3' side. LinGT2-2D has a probe region corresponding to the second domain as well as a Tag sequence 5'-GAGGTGTGGTGGAGAGGAGGTGTG-3' (SEQ ID NO: 24) as shown in Example 3.

Among these probes, the Tag sequences of 95-42UD and LinTG3-5 are complementary to each other so that an arm structure can be formed by complementary double strand formation by adding LinTG3-5 to the carrier on which 95-42UD is immobilized, which results in a structure in which the first domain recognizing part and the second domain recognizing part are adjoined via the arm part. Further, the Tag sequences of 95-25UD and LinGT2-2D are complementary to each other so that an arm structure can be formed by complementary double strand formation by adding them into a liquid phase, which results in a structure in which the first domain recognizing part and the second domain recognizing part are adjoined via the arm part.

In the same manner as in Example 1, 95-42UD alone was immobilized onto carboxy beads. Further, biotin-labelled amplicons were prepared by PCR amplification with biotin-labelled primers using plasmid DNAs in which individual genes were incorporated, as a template.

The biotin-labelled amplicons obtained for individual genes were hybridized with the beads onto which 95-42UD was immobilized, under hybridization conditions. Here, LinTG3-5 which forms a complementary double strand with the immobilized probe 95-42UD was allowed to coexist in the liquid phase to form a probe structure in which the first domain and the second domain were adjoined. Further, as a competitor, 95-25UD alone, LinGT2-2D alone, or a combination of 95-25UD and LinGT2-2D was allowed to coexist. Streptavidin-phycoerythrin (a fluorescent substance) was allowed to react herein and the fluorescence intensity remaining on the beads after washing was measured and its relative values are shown in Table 8.

**Table 7**

| | Number of mismatches | |
|---|---|---|
| | In the first domain | In the second domain |
| *130101 | 0 | 0 |
| *130201 | 0 | 2 |
| *1403 | 2 | 2 |

Table 7 shows the number of mismatches in the sequences of the first domain and the second domain between the probe used and the individual DRB1 genes. The sequences of the probe regions used in this example are completely matched with DRB1*130101 gene in both two domains but mismatches are present in the second domain with DRB1*130201 and in the first and the second domains with DRB1*1403.

**Table 8**

| Oligonucleotide immobilized | 95-42UD | | | |
|---|---|---|---|---|
| Oligonucleotide added | LinTG3-5 | | | |
| Competitor oligonucleotide | 95-25UD and LinGT2-2D | 95-25UD | LinGT2-2D | - |
| Plasmid DRB1*130101 | 11696 | 10828 | 10887 | 11091 |
| Plasmid DRB1*130201 | 303 | 653 | 1074 | 1152 |
| Plasmid DRBI*1403 | 25 | 12 | 37 | 39 |

Table 8 shows the fluorescence intensity remaining on the beads after hybridization using the biotin-labelled amplicons prepared from the individual template DNAs. In the case where no competitor was added, a high florescence value presumably due to cross-hybridization was obtained with DRB1*130201. On the other hand, in the case where a competitor capable of complementary double strand formation was added, the fluorescence value with DRB1*130201 was markedly decreased as compared to that without the addition, which confirmed that the use of the competitor significantly suppressed the cross-hybridization.
Namely, when a probe for detecting nucleic acid of the present invention is used as a competitor, cross-hybridization can be suppressed and the recognition accuracy of the detecting probe can be improved.

## Claims

1. A nucleic acid fragment set comprising a plural number of nucleic acid fragments capable of individually hybridizing with a plural number of target sequences, wherein
each nucleic acid fragment has a region ligatable with each other, and
the affinity between such ligatable regions is adjusted to a higher level than the affinity between the target sequence and the nucleic acid fragment.

2. The nucleic acid fragment set according to claim 1, wherein the regions ligatable with each other consist of nucleic acids.

3. The nucleic acid fragment set according to claim 1 or 2, wherein at least one of the plural number of nucleic acid fragments is immobilized on a solid phase.

4. The nucleic acid fragment set according to claim 3, wherein the solid phase is in a bead shape or in a plane shape.

5. The nucleic acid fragment set according to any one of claims 1 to 4, wherein when the plural number of target sequences are present on the same nucleic acid strand, it is hybridizable with said nucleic acid strand.

6. A nucleic acid fragment, for use in the nucleic acid fragment set of any one of claims 1 to 5.

7. A probe for detecting nucleic acid, consisting of the nucleic acid fragment set of any one of claims 1 to 5.

8. The probe according to claim 7, for use in detecting a gene selected from the group consisting of human leukocyte antigen (HLA) genes, T-cell receptor genes, red blood cell group determining genes, and Rh antigen genes.

9. A competitor for detecting nucleic acid, consisting of the nucleic acid fragment set of any one of claims 1 to 5.

10. The competitor according to claim 9, wherein the affinity between a target sequence and a nucleic acid fragment is adjusted to be lower than or equivalent to the affinity between the nucleic acid fragment used in the probe of claim 7 or 8 and the target sequence.

11. A method for detecting a nucleic acid of interest in which a plural number of target sequences are capable of presenting on the same nucleic acid strand, comprising the steps of
(a) bringing the probe for detecting nucleic acid of claim 7 or 8 into contact with a nucleic acid sample under hybridization conditions, and
(b) assessing the presence of the nucleic acid of interest in the nucleic acid sample by whether or not the probe and the nucleic acid sample are hybridized.

12. The method according to claim 11, wherein the competitor for detecting nucleic acid of claim 9 or 10 is used in step (a).

13. The method according to claim 11 or 12, further comprising the step of amplifying the nucleic acid of interest in the nucleic acid sample prior to step (a).

14. The method according to claim 11, 12, or 13, wherein the nucleic acid of interest is selected from the group consisting of human leukocyte antigen (HLA) genes, T-cell receptor genes, red blood cell group determining genes, and Rh antigen genes.

15. A kit for detecting nucleic acid, at least comprising the probe for detecting nucleic acid of claim 7 or 8 and a competitor for detecting nucleic acid of claim 9 or 10.

16. The kit for detecting nucleic acid of claim 15, further comprising a primer for amplifying the nucleic acid of interest.

17. Use of the nucleic acid fragment set of any one of claims 1 to 5 for detecting a nucleic acid of interest in which a plural number of target sequences are capable of presenting on the same nucleic acid strand.
